# EUROPEAN PATENT SPECIFICATION

(11) **EP 0 414 741 B1**
(45) Date of publication and mention of the grant of the patent: **15.03.1995**
(21) Application number: 89905144.5
(22) Date of filing: 21.04.1989
(51) Int. Cl.: A61K 39/395, A61K 39/44, A61K 45/05

(54) **FURTHER IMPROVEMENTS RELATING TO DRUG DELIVERY SYSTEMS**
VERABREICHUNGSSYSTEME FüR ARZNEIMITTELN
NOUVELLES AMELIORATIONS RELATIVES A DES SYSTEMES D'ADMINISTRATION DE MEDICAMENTS

(30) Priority: 22.04.1988 GB 8809616
(43) Date of publication of application: 06.03.1991
(60) Divisional application: 94113490.0
(73) Proprietor: CANCER RESEARCH CAMPAIGN TECHNOLOGY LIMITED, London NW1 4JL (GB)
(72) Inventor: BAGSHAWE, K., c/o Charing Cross Hospital, CRC Lab., London W6 8RF (GB); ROGERS, G., c/o Charing Cross Hospital, CRC Lab., London W6 8RF (GB); SHARMA, S., c/o Charing Cross Hospital, CRC Lab., London W6 8RF (GB)
(74) Representative: Goldin, Douglas Michael
(86) International application number: GB8900427
(87) International publication number: WO8910140

(56) References cited:
- EP-A- 89 880
- EP-A- 140 728
- EP-A- 186 551
- WO-A-88/07378

## Description

THIS INVENTION relates to methods and systems for the control of neoplastic cell growth and is particularly concerned with methods and systems involving the localisation of cytotoxic agents at tumour sites.

In our earlier Patent WO 88/07378 we disclose a two-component system which comprises
(i) a first component (Component A-E) that is an antibody fragment capable of binding with a tumour associated antigen, the antibody fragment being bound to an enzyme capable of converting a prodrug into a cytotoxic drug;
(ii) a second or final component (Component PD) that is a prodrug convertible under the influence of the enzyme to a cytotoxic drug (CD).

In our earlier Patent and in this present Patent Application, the word 'tumour' is to be understood as referring to all forms of neoplastic cell growth including carcinomas, sarcomas, lymphomas and leukaemias.

Our existing system is used to target cytotoxic prodrugs to the site of neoplastic cell growth. However, although this two-component system is useful in the control of tumours, the amount of first component that localises per gram of tumour in humans may be less than 0.1% the total amount of first component administered. A substantial proportion of the non-localised first component including active enzyme remains in the circulating blood. It is therefore desirable to reduce the amount of this freely circulating antibody-enzyme first component before administration of the prodrug so as to limit the formation of active drug in blood from where it would be carried to normal tissues. A similar problem arises when the first component is prepared from whole antibody.

The present invention provides methods for the removal from blood of residual conjugates of the enzyme or for inactivation of the enzyme in the residual first component with whole antibody or antibody fragment or equivalent component after the first component has localised at tumour sites.

The present invention is concerned with reducing problems arising in the clinical use of our above-mentioned two-component system from the presence in the patient of non-localised first component and permits further extension to be made to the nature of the first component.

The present invention provides a three-component system, for use in association with one another, in the treatment of malignant diseases characterised by malignant cells, comprising: a first component which is a substance or conjugation of substances characterised by (a) one or more molecular configurations that are complementary to molecular configurations associated with malignant cells, such that the first component localises selectively at sites of malignant cells and (b) additionally by one or more catalytic sites; a second component which is able to bind to such part of the first component so as to inactivate the catalytic site of the first component and/or accelerates the clearance of the first component from the blood when the first and second components are administered clinically; a third component which is a substrate for the catalytic site on the first component, one of the end products of the catalysis being a substance which is more cytotoxic to maligant cells than the third component.

The clinically most useful form of the first component is a conjugate of an antibody or fragment thereof and an enzyme while the clinically most useful form of the third component is a prodrug convertible under the influence of the enzyme activity of the first component into a cytotoxic compound. The antibody will desirably be one recognising and binding preferentially to a tumour associated antigen and it will be apparent to those skilled in the art how to match the antigen associated with the particular tumour to be treated with the antibody or fragment to be used in the first component, how to match the cytotoxic compound to the tumour to be treated and how to match the prodrug to the enzyme activity of the first component.

As described in our Patent WO 88/07378 the prodrug can be benzoic acid mustard glutamide that converts to benzoic acid mustard [p-(bis-2-chloroethyl)amino]benzoic acid under the influence of a carboxypeptidase. However, the principles of this invention are equally applicable to other prodrugs releasing benzoic acid mustard or analogues thereof or other cytotoxic drugs using enzymes appropriate to the removal from the prodrug of the structural feature distinguishing the prodrug from the cytotoxic drug.

When antibody is used in the first component, it can be whole antibody or one of the antibody fragments, e.g. F(ab')₂ or other fragment as described in our above-mentioned earlier filed International Patent Application. The function of the antibody in the first component is to assist in the localisation of the first component in the region of the tumour to be treated and this function can also be fulfilled by substances other than antibodies, e.g. hormones or growth factors that have affinity to other tumour-associated compounds.

In one embodiment of the system of the invention the first Component is a conjugate of an antibody to a tumour-associated antigen or a fragment thereof that includes the antigen binding site of the antibody, said antibody or fragment thereof being conjugated directly, or indirectly through a linking component, to an enzyme or to an antibody or antibody fragment with catalytic functions. In this case the conjugation can be effected by chemical bonding or by splicing together nucleic acid sequences that code at least for one or more antigen binding sites and one or more catalytic sites and such other sequences as are necessary to retain the vector function of the molecule and the catalytic function of the peptide when the gene product of the reconstructed nucleic acid sequence is expressed by eukaryotic or prokaryotic cells.

In a further embodiment, the antibody in the first component is bivalent and formed by bonding together two univalent antibody fragments, or by recombinant DNA techniques, one fragment having affinity for a tumour marker substance, the other having affinity for an enzyme. In such a case the conjugate can be formed either in vitro prior to administration or in vivo by first administering the bivalent antibody, allowing time for it to localise at tumour sites and then administering the enzyme for capture by the second arm of the antibody localised at tumour sites.

The antibody of the first component may be a human immunoglobulin, or fragment thereof, having antigen binding site(s) of human origin or having antigen binding site(s) of non-human species.

Reichmann L, Clark M, Waldmann H, and Winter G (Reshaping human antibodies for therapy - Nature 332: 323-327, 1988) shows that by genetic engineering techniques the antigen binding sites of a rodent monoclonal can be incorporated into human immunoglobulin fragments so that the immunogenicity of the molecule in the human subject is minimised. It has been shown that immunoglobulin-gene DNA can be manipulated so that the Fc portion of the antibody has been replaced with an active enzyme moiety (Neuberger MS, Williams GT, Fox RO - Nature 312: 604-608, 1984) and such genetically engineered constructs bearing one or more antigen binding sites and one or more enzyme active sites can be used in the present invention.

It has been observed that when monoclonal antibodies derived from one species are injected into another species the host antibody response may be (at least partially) directed at the idiotype of the injected monoclonal. (Rowe et al, IRCS Med Sci. 13: 936-7, 1985). Similarly, it is well-known that bacterial products, including enzymes, are immunogenic in mammalian species including man.

The present system will be most effective in man and suitable for repetitive use when the immunogenicity of a first component antibody-enzyme conjugate is minimised or if immune tolerance to such conjugates has been induced. This is likely to be achieved through genetic engineering methods since the production of monoclonals to specific antigens by human hybridomas has so far proved difficult to achieve consistently. It has been shown that the antigen binding site of a rodent monoclonal antibody can be incorporated into a human immunoglobulin framework (Reichmann et al, Nature 332: 323-327, 1988).

It has also been shown that antibodies can be produced which function as enzymes (Pollack SJ et al, Science 234, 1570-1573, 1986) so the ultimate form of the antibody-enzyme conjugate may be a human immunoglobulin construct expressing one or more antigen binding sites characterised by peptide sequences of non-human origin and one or more catalytic sites.

With a 'humanised' antibody conjugated to a human enzyme or a non-human enzyme which has been rendered non-immunogenic in man, or a construct with both antigen binding and catalytic sites on a human immunoglobulin, the second component of our system will need to be directed at either the active site of the enzyme or at the idiotype of the antibody since a clearing antibody against the generality of human immunoglobulins would be unsuitable.

We have developed several different methods of removing what becomes the unwanted circulating first component after maximum localisation of the first component has occurred in the region of the tumour to be treated. The exact nature of the second component will depend upon the particular strategy to be used for removal of non-localised first component but the second component will always be one that either inactivates the catalytic site in the first component and/or accelerates its clearance from the blood.

According to one embodiment the second component is an antibody or fragment thereof having an affinity for an antigen binding site of an antibody of the first component or the active site of an enzyme of the first component or another constituent part of the first component.

According to a further embodiment the second component is one causing rapid loss of enzyme activity of the first component in plasma without incurring significant loss of enzyme activity from tumour sites.

According to a further embodiment the second component includes covalently bound galactose residues or covalently bound residues of other sugars such as lactose or mannose, so that it can bind enzyme in plasma but be removed together with the enzyme or antibody-enzyme conjugate from plasma by receptors for galactose or other sugars in the liver in a period of time such that the antibody does not enter the extravascular space of the tumour in an amount sufficient to inactivate tumour localised enzyme. In this case, galactose residues in the second component are either chemically added or exposed by removing terminal sialic acid residues.

Terminal sialic residues play a role in maintaining the presence of glycoproteins in the blood. Removal of terminal sialic acid by neuraminidase exposes proximal sugar residues such as galactose. Desialylated proteins are rapidly removed from the blood by receptors in liver and possibly other sites. (Morell et al, J.Biol. Chem. 246: 1461-1467, 1971).

Asialo human chorionic gonadotrophin was prepared by digesting 1 mg of a glycoprotein in 1 ml of 0.05M sodium acetate buffer, pH 5.6, containing 0.15M NaCl with 20ug of neuraminidase (Sigma Type II from Vibro cholerae) at 37°C for 30 minutes. The neuraminidase was then removed. Sialo and Asialo preparations were compared for clearance in A2G mice. T 1/2 for the sialyted hCG was in excess of 24 hours but T 1/2 for the desialyted form was <5 min.

A further embodiment of the invention is one wherein the first component is an antibody enzyme conjugate modified by addition of, or exposure of, additional galactose or other sugar residues, and is for administration in conjunction with asialofetuin, which has sufficient affinity for the corresponding sugar receptors which are involved in removing galactosylated or similar proteins from the blood, to form an asialofetuin blockade.

This embodiment requires the addition of galactose residues to the antibody-enzyme conjugate by methods similar to those described for galactosylating the clearing antibody. Before administering the galactosylated conjugate the galactose receptors are blocked by an agent which binds more avidly to those receptors than the galactosylated conjugate. This results in maintenance of a high level of conjugate in the plasma until galactose receptors are again free to take up the conjugate.

Asialofetuin is a substance known to bind strongly to galactose receptors but other less immunogenic substances could be identified or developed for the same purpose.

A further embodiment of the invention is one wherein the second component is conjugated to a macromolecule such as a dextran, liposome, albumin microsphere or macroglobulin with a molecular weight in excess of 500,000 Daltons or a biodegradable particle such as a blood group 0 erythrocyte so that the second component is restrained from leaving the vascular compartment by virtue of the size of the conjugate.

A further embodiment of the invention is one wherein the second component is an antigen, hapten or protein construct bearing an epitope capable of binding with the first component to form complexes having accelerated clearance from plasma.

In a still futher modification of the system, the first component is covalently linked to biotin or derivatives of biotin, and the second component then comprises the biotin-binding glycoprotein avidin found in egg white, or streptavidin, itself optionally covalently linked to galactose.

Biotin may be conjugated to antibody or fragment thereof by reaction with a 10 molar excess of sulphosuccinimidyl 6-(biotinamido)hexanoate at pH 8.5 at 4°C for 16 hours. The product is purified by chromatography on Sephadex G-25. (Sephadex is a Registered Trademark).

In all these cases, except for the procedure involving asialofetuin, the second component may be conjugated with a macromolecule or biologically degradable particle such that the additional component does not, to any appreciable extent, escape from the vascular compartment. The macromolecule may be optionally galactosylated.

Macromolecules to limit clearing component escaping from the vascular compartment are likely to be in excess of 500,000 Daltons and include carbohydrates such as dextrans, lipids as in liposomes or proteins such as in albumin microspheres or a macroglobulin. An example of a biodegradable particle for this purpose is an erythrocyte of blood group 0.

As an alternative to basing the first component on an antibody it may be based on a hormone or growth factor or substance other than an antibody and for which receptors capable of binding that substance exist on a tumour.

Tumours may express receptors for growth factor hormones and other metabolites such that these can be used as target sites for selective delivery. The corresponding growth factor, hormone, metabolite or genetic construct might then be used as the vector to carry an enzyme to tumour sites in a comparable fashion to antibody. There are literature examples of radiolabelled hormones, growth factors and metabolites localising in tumours (Krenning et al, Lancet i 242-244, 1989 (Somatostatin); Hattner et al, Am. J. Roentgenol. 143: 373-374, 1984) but in none of these were the vectors used to convey enzyme to tumour sites.

The enzyme part of the first component can be of human or non-human origin. The advantage of using an enzyme of human origin lies in avoiding or minimising the immunogenic effect of an enzyme of non-human origin. The disadvantage of an enzyme of human origin is the probability that the presence of enzyme in human tissues will activate the prodrug, thus releasing active drug at the non-tumour sites. However, it may be possible to identify certain human enzymes which are so distributed that this activation would not cause a serious problem. Also, inactivation of such enzymes in tissues might be achieved by using high affinity anti-enzyme antibody fragments which would be rapidly cleared from the plasma before giving the first component conjugate. Where the human enzyme is normally present in the plasma, this would activate prodrug in the plasma which would be highly disadvantageous and be liable to cause general toxic effects. Administration of an appropriately selected antibody or antibodies or fragments directed at the enzyme in the conjugate would however also have the effect of inactivating free, naturally recurring enzyme of the same type in the plasma. In the case of human phosphatases there are several different forms produced in different tissues but there is little evidence of specificity for substrates. There is also evidence that antibodies directed at one isotype of alkaline phosphatase may bind to other isotypes.

The immunogenicity of an enzyme of non-human origin may be reduced by modification of its amino acid sequence.

In order to render the antibody-enzyme conjugate less immunogenic, it can be modified by conjugation to polyethylene glycol or other polymers, e.g. by reaction with the cyanuric chloride derivative of methoxypolyethylene-glycol 5000. The resulting material may be employed directly, or may be pre-injected to render the host tolerant to further injections of the native conjugate. Reaction with synthetic copolymers of D-glutamine acid and d-lysine or with tri-peptidyl-modified organic polymers comprising alternate D-glutamic acid and D-lysine on the exterior ends of the side chains can be predicted to depress the immunogenicity of the conjugate. See, for example, Abuchowsky A., van Es T., Palezuk NC, Davis FF - J. Biol. Chem. 252: (11), 3578-81, 1977, or Kawamura K, Igarishji, T, Fujii T., Kamasaki J., Wada, H., Kishimoto, S. Int. Arch. Allergy appl. Immunol. 76: 324-330, 1985.

To minimise clinical problems arising from the use of immunogenic antibody enzyme conjugates and immunogenic antibodies or avidin-like constructs, it is desirable to minimise or delay the production of host antibodies to xenospecific proteins by using immunosuppressive agents such as cyclosporin, cyclophosphamide, methotrexate, azathioprine etc., in order to provide sufficient time for the delivery of repeated treatments.

The ability of cyclosporin to prevent antimurine antibody responses by rabbits and in patients has been demonstrated. See, for example, Ledermann, JA. Begent, RHJ. Bagshawe, KD. Br. J. Cancer, 58: 562-566, 1988, or Ledermann, JA. Begent, RHJ. Riggs, SJ. Searle, F. Glaser, MG, Green, AJ. Dale, RG. Br. J. Cancer 58: 654-657, 1988.

In certain clinical conditions, it can be advantageous for the first component to be conjugated to a signal producing molecule such as a radioisotope suitable for scintigraphic imaging by gamma camera so as to confirm localisation of the first component at tumour sites.

Radiolabelling can be achieved with ¹²⁵I or ¹³¹I with standard methods either using chloramine T (Greenwood F, Hunter W, Glover JS, Biochem. J. 89: 114-123, 1963, Fraker PJ, Speck JC, Biochem. Biophys. Res. Comm. 80: 849-857, 1978), but other methods of iodination or radiolabelling with other isotopes such as indium or technetium can also be used. Such radiolabelled conjugates are generally used in clinical practice in amounts required for radioimmunolocalisation by immunoscintigraphy and would generally form only a small part of the administered conjugate.

Modern methods of analysis may be used in conjunction with a radiolabelled fraction of the conjugate to determine the concentration of the conjugate at target sites and non-target sites and thus help determine the optimum time for administration of the prodrug. (Riggs et al, Int. J. Cancer Supp. 2, 95-98, 1988, Dewhurst et al, (Abstract) Br. J. Cancer 1988).

The system of the present invention can include more than one type of first component and/or more than one type of second component and/or more than one type of third component.
Heterogeneity in expression of target antigens and receptors on cells in tumours may require the use of more than one vector to carry enzyme to tumour sites. Multiple vectors may permit greater or more economical delivery of enzyme to tumour sites. It may also be advantageous to use more than one type of prodrug to generate a state equivalent to multidrug chemotherapy so as to reduce the risks of drug resistance and this in turn may require the use in treatment of more than one type of enzyme. These variations may in time require the use of more than one second component to achieve the required clearing of enzymes from plasma and other non-tumour sites.

The three components forming the system of the present invention are designed to be used in association with one another in a method of treatment of the human or animal body by therapy.

It is specifically designed for use in a method for the treatment of malignant diseases including carcinomas, sarcomas, lymphomas and leukaemias which comprises administering to a host in need of such treatment an effective amount of a system.

In such a method, the first component is administered first, the second component is administered subsequent to the first component after a time interval such that the first component has selectively localised at the site of malignant cells and the third component is administered subsequent to the second component after a time interval such that the concentration of the first component in the blood has reduced from its peak value.

The following Examples are given to illustrate various aspects of the invention.

### EXAMPLE 1

This is to illustrate the inactivation of an active enzyme site by an antibody.

A monoclonal antibody (SB43) was produced by conventional methods following immunisation of the lymphocyte donor mouse with carboxypeptidase G₂. Microtitre plates were coated with three units per well of carboxypeptidase G₂ and incubated with supernatants from the hybridoma culture, and it was found that ¹²⁵ Iodine labelled rabbit anti-mouse antibody bound to the coated wells with a 50% binding titre at a dilution of the supernatant of 1:800 in buffered solution. Assay of enzyme activity was assessed after 24 hours incubation at 37°C in buffer containing a 1000-fold dilution of the antibody (hybridoma supernatant). Enzyme incubated with buffer alone for 24 hours retained most of its capacity to cleave methotrexate as shown by optical density measurements (54.1 carboxypeptidase units/ml initially falling to 40 units/ml activity after 24 hours). In the wells containing the antibody (hybridoma supernatant) the activity was reduced to 13.0 carboxypeptidase units/ml. The antibody alone had no effect on the optical density of methotrexate. These experiments show that the enzyme active site on the carboxypeptidase can be substantially inactivated by an antibody raised against the enzyme. Monoclonal antibodies to carboxypeptidase G₂, raised by the technique described above will only have a similar enzyme inhibiting property if they are directed at epitopes in or close to the active site of the enzyme.

### EXAMPLE 2

### Evidence for localisation of antibody-enzyme conjugate at tumour sites

1. 4 nude mice bearing LS174T human colon cancer xenografts on their L flanks were injected with A5B7 (Fab')₂ monoclonal antibody directed at carcinoembryonic antigen conjugated to carboxypeptidase G2 and labelled with ¹²⁵I. An immunoscintigraph taken after 48 hours confirms localisation of the conjugate at the tumour sites.
   Similar results were obtained using the following conjugates:
2. A5B7 intact IgG - carboxypeptidase
3. A5B7-F(ab')₂ - nitroreductase
4. SB10 (antiHCG) -F(ab')₂ - carboxypeptidase

### 2. METHODS OF CONJUGATION OF ANTIBODY TO ENZYME

Conjugation of IgG or F(ab')₂ with carboxypeptidase was accomplished by mixing a maleimide derivative of the enzyme with a thiolated antibody.

### 1) Thiolation with S-acetylthioglycolic acid N-hydroxysuccinimide ester (SATA).

IgG or F(ab')₂ in 0.1M sodium phosphate buffer, pH 7.6, (containing 372 mg of EDTA/litre) at 1-2 mg/ml was treated with a 15 molar excess of SATA (made up 20 mg/ml in DMF) and left at about 20°C for approximately 2 hours. The thiolated antibody was then passed down a column of Sephadex® G-25 to remove excess SATA. The thiol was deacetylated by adding 0.1 volumes of 3.5% hydroxylamine, pH 7.5, prepared by adding disodium hydrogen phosphate to an aqueous solution of hydroxylamine hydrochloride.

### 2) Thiolation with N-succinimidyl 3-(2-pyridyldithio)propionate (SPDP).

IgG or F(ab')₂ in 0.2M sodium phosphate buffer, pH 8.6, at a concentration of 4 mg/ml was treated with a 15 molar excess of SPDP in ethanol and left at r.t. for 1 hour. Excess SPDP was removed on a column of Sephadex® G-25 equilibrated in 0.1M sodium acetate buffer, pH 4.5. The pyridyldisulphide group was then reduced for 30 minutes with 50 ul/ml of 100 mM dithiothreitol and excess reducing agent removed by Sephadex® G-25 gel filtration.

### 3) Derivatisation of carboxypeptidase.

Carboxypeptidase in 0.1M sodium phosphate buffer, pH 7.6 (containing 372 mg EDTA/litre) at 2 mg/2.5 ml was treated for 3 hours with a 15 molar excess of succinimidyl 4-(p-maleimidophenyl)butyrate (SMPB) dissolved in THF. The excess SMPB was removed by gel filtration on Sephadex® G-25.

### 4) Conjugation

The derivatised enzyme was mixed with an equimolar amount of thiolated antibody and the progress of the conjugation monitored by gel filtration. When no further reaction was judged to take place the mixture was concentrated and the conjugate purified by gel filtration. Typical enzyme activities obtained were 150-200 units/mg of conjugate.

### EXAMPLE 3

### Evidence that SB43 binds to carboxypeptidase G2 in vitro.

Microtitre wells were coated with carboxypeptidase G2 and monoclonal antibodies SB43 (raised to carboxypeptidase G2) and SB10 (raised to human chorionic gonadotrophin) were added in dilution and incubated before aspiration. ¹²⁵I anti-mouse IgG was then added and incubated for 30 minutes followed by aspiration and washing. The wells were cut out and counted in a gamma counter. The results set out below show no significant binding of SB10 to the carboxypeptidase coated wells but all dilutions of SB43 used showed high counts indicating binding to the carboxypeptidase. SB43 modified by addition of galactose moieties was included a similar dilution and showed similar binding to unmodified SB43.

The microtitre plate was coated with 0.1 »g. CPG₂ per well and incubated overnight with SB43. The ¹²⁵I-mouse IgG was then introduced, the plate incubated for 1 hour, washed and radio-counted.

| SAMPLE | TIME | COUNTS(1) | CPM(1) | %CV | |
|---|---|---|---|---|---|
| 1 | 30 | 1092 | 2171.4 | 3.1 | (Negative Control-SB10-Anti-hCG) |
| 2 | 30 | 805 | 1588.6 | 3.6 | (Negative Control-SB10-Anti-hCG) |
| 3 | 30 | 16428 | 33278.0 | .8 | SB43 x 20 |
| 4 | 30 | 23339 | 47302.9 | .7 | SB43 x 20 |
| 5 | 30 | 22020 | 44679.3 | .7 | SB43 x 100 |
| 6 | 30 | 22096 | 44833.8 | .7 | SB43 x 100 |
| 7 | 30 | 8437 | 16873.4 | 1.1 | SB43 x 1000 |
| 8 | 30 | 7671 | 15336.8 | 1.1 | SB43 x 1000 |
| 9 | 30 | 15411 | 31052.2 | .8 | SB43-Gal-10x20 |
| 10 | 30 | 15418 | 31066.3 | .8 | SB43-Gal-10x20 |

### EXAMPLE 4

### Evidence that SB43 inactivates/clears Ab-E-conjugate in vivo from plasma

The level of carboxypeptidase G2 activity in plasma can be monitored by observing the hydrolytic cleavage of methotrexate, a folic acid analogue, to pteroates and L-glutamate. When a conjugate of A5B7-F(ab')₂-carboxypeptidase G2 (25 enzyme units) was injected intravenously and plasma samples obtained 20 hours later significant hydrolysis of methotrexate was observed equivalent to 1.12 to 1.45 enzyme units/ml as shown by the steps of the spectrophotometric print-out.

Mice which were injected with galactosylated anti-carboxypeptidase (SB43-Gal 10) 19 hours after A5B7F(ab')₂-CPG2 and plasma taken 5 minutes and 15 minutes later caused no significant hydrolysis of methotrexate showing that the enzyme had been inactivated and/or cleared from the plasma.

### EXAMPLE 5

### Biotinylation of antibody-enzyme conjugate

Carboxypeptidase G2 (44.4 mg) in 0.05M sodium bicarbonate buffer, pH 8.5 (1.5 ml) was mixed with sulphosuccinimidyl 1-6-(biotinamide) hexanoate (292 ug in 73 ul of buffer and left at room temperature for 3 hours. The enzyme was then separated on Sephadex® G-25 equilibrated in 0.15M sodium phosphate buffer pH 7.6, containing 372 mg EDTA/litre and the volume adjusted to 2.5 ml. The biotinylated enzyme was then treated for 3 hours with a 15 molar excess of succinimidyl 4 - (p-maleimido phenyl)butyrate (SMPB) dissolved in tetrahydrofuran and the excess SMPB removed by gel filtration on Sephadex® G25. The derivatised enzyme was then conjugated to thiolated F(ab')₂ fragment of the A5B7 antibody as described in Example 2.

Affinity purified avidin was used as obtained from Sigma Ltd., 10-15 units/mg protein. Mice received 20 ug of biotinylated A5B7-carboxypeptidase G2 conjugate followed after one hour by avidin in the dose range 20-500 ug. Rapid clearance of the enzyme activity in plasma was observed comparable to that observed with SB43 monoclonal antibody in Example 4.

### EXAMPLE 6

IgG class immunoglobulins carrying different specificities on their two binding sites can be made by a fusion technique employing hybridomas producing different antibodies (Milstein C & Cuello AC. Nature 305: 537-540, 1983; Sfaerz UD & Bevan MJ. Proc. Nat. Acad. Sci. USA 83: 1453-1457, 1986) or by chemical conjugation of univalent preparations of each of the antibodies required as used here. F(ab')₂ fragments of monoclonals SB10 (anti-human chorionic gonadotrophin (anti-hCG)) and A5B7 (anti-carcinoembryonic antigen, (anti-CEA)) were reduced in the presence of arsenite. F(ab')₂ fragment (20 mg) in 0.1M sodium phosphate buffer pH 7.6 (10 ml) was mixed with sodium arsenite (12.4 mg) EDTA (3.72 mg) and 2-mercapto-ethylamine (1.13 mg) and left at room temperature. Solid 5,5'-dithio-bis-(2-nitrobenzoic acid) (19.8 mg) was added and the mixture left at about 20°C for 18 hours. The thionitrobenzoate modified Fab' (TNB derivative) was purified by gel filtration on Sephadex G-25 with a yield of approximately 70% based on protein recovery.

The TNB derivative of anti-CEA (4.8 mg) in 5 ml of 0.1M sodium phosphate buffer, pH 6.8, containing 1mM EDTA was treated for 30 minutes with mercaptoethylamine to give a final concentration of 10mM. The reduced TNB-anti-CEA Fab' was then purified by gel filtration on Sephadex G-25 equilibrated in 0.1M sodium phosphate buffer pH 7.0, containing 1mM EDTA. The reduced TNB-anti-CEA Fab' was then incubated with 4.9 mg (5 ml) of TNB derivative of anti-hCG for 16 hours and the formation of bispecific antibody monitored by gel filtration on a Superose S-12 column (Pharmacia). The yield was 20% based on protein after purification of the bispecific antibody on the Superose S-12 column. (Superrose is a Registered Trademark) The ability of this ¹²⁵I labelled biospecific antibody to function in vivo and bind to its corresponding antigen was demonstrated by injection into nude mice bearing either CEA producing LS174T tumours or hCG producing CC3 tumours. At 20 hours post-injection mean tumour to organ ratios were:

| anti CEA/ anti hCG | | non-specific F(ab')₂ |
|---|---|---|
| blood | 2.9 | 0.6 |
| liver | 3.9 | 1.9 |
| kidney | 1.8 | 1.2 |
| lung | 3.2 | 1.2 |
| spleen | 6 | 3.0 |
| colon | 9 | 5.3 |

(Conjugation of A5B7 and SB43 (anticarboxypeptidase) has not yet been performed but above experiment demonstrates retention of binding site function).

### EXAMPLE 7

### Method for galactosylation

Cyanomethyl 2,3,4,6-tetra-0-acetyl-1-thio-b-D) galactopyranoside (400 mg) in anhydrous methanol (10 ml) was treated with 5.4 mg of sodium methoxide in 1 ml of anhydrous methanol at about 20°C for 48 hours. A stock solution of IgG in 0.25M sodium borate buffer, pH 8.5 at 1.3 mg/ml was prepared. Since the number of galactose residues conjugated to IgG was not determined, a unitage was adopted corresponding to the number of microlitres of the activated galactose derivative added to 200 ug of IgG at a concentration of 1.3 mg/ml.

Aliquots of the activated galactose derivative (e.g. 300, 80, 40, 10, 5 and 2 ul) were dispensed into 3 ml glass ampoules and evaporated to a glassy residue in a stream of nitrogen under vacuum. 200 micrograms of IgG (153 ul of stock solution) were added to each aliquot and mixed until the residue was dissolved. After 2 hours at about 20°C the solution was dialysed against 3 changes of PBS (phosphate buffer saline). Tests were performed to determine what level of galactosylation gave the most effective results. It was found that, in terms of the unitage defined above, 10 ul of the activated glactose derivative added to 200 ug of IgG gave the most satisfactory results. Figure 1A shows blood and tumour levels up to 48 hours after injection of monoclonal SB43 (anti-carboxypeptidase). A, native form; B, galactosylated form. Further studies were performed, each in groups of 4 mice, bearing LS174T tumours and receiving A5B7 F(ab')2-CP (carboxypeptidase G2) conjugate followed after 24 hours by SB43 (anti-carboxypeptidase) galactosylated to the 10 ul level (as defined previously) or with saline as control followed one hour later by the bis-chloro benzoic acid mustard prodrug, 4-[bis-(2-chloroethyl)amino]-benzoic acid glutamide. Mice were killed at intervals following administration of the prodrug, the tissues extracted and prodrug and active drug levels were measured by HPLC methods.

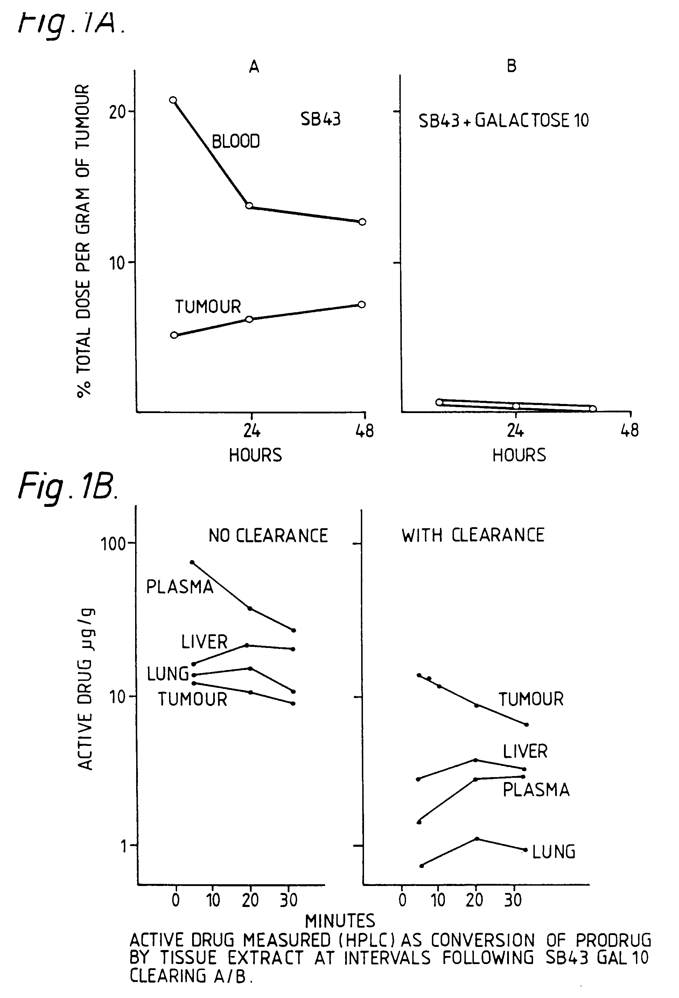

Figure 1B shows the levels of active drug in various tissues in mice which had not received SB43-Gal 10 clearing antibody with those that had. In the absence of SB43-Gal 10 clearing antibody, levels of active drug were significantly lower than those found in liver and lung but in animals receiving the SB43-Gal 10 clearing antibody tumour levels were higher than in any other tissue.

As part of the same experiment two groups of mice, one with and one without SB43-Gal 10, were killed without receiving the prodrug. The tissues were extracted and tested for ability to convert prodrug to active drug in vitro, The results are shown in the Table and expressed as percentage of injected dose of carboxypeptidase per gram of tissue.

| In vivo admin | Carboxypeptidase G2% i.v. dose per gram of tissue at 48 hours | | |
|---|---|---|---|
| | Tumour | Plasma | T/P |
| Ab-CPG2 | 8.1(+0.69) | 0.22 | 36 |
| AB-CPG2 + Gal 10 antiCPG2 24 hours later | 7.2(+1.42) | 0.026 | 277 |

### EXAMPLE 8

Where the antigen corresponding to an intravenously administered antibody is present in the blood, antigen-antibody complexes form and these accelerate clearance of the antibody from the circulation into the reticuloendothelial cells. Accelerated clearance of anti-hCG antibodies W14 and SB10 occurs when these are injected into nude mice bearing CC3 hCG secreting tumours when compared with A5B7 anti-CEA antibody in LS174T bearing mice which express CEA on LS174T cell membranes but do not secrete CEA into the blood.

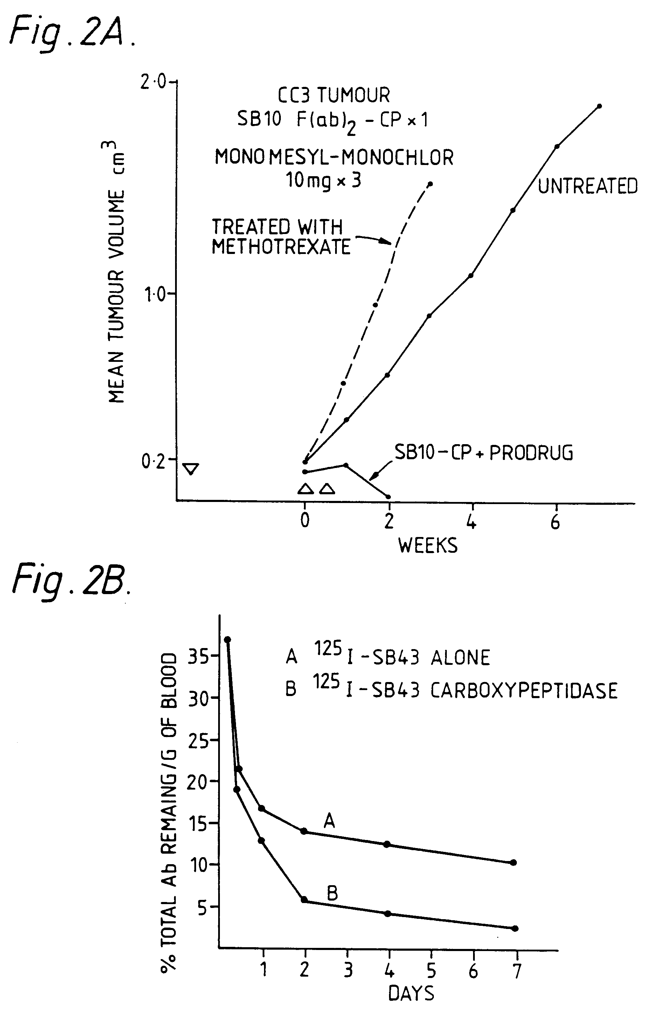

Figure 2A shows the result of administering SB10 F(ab')₂-CP (50 units CP) intravenously to 6 CC3 bearing nude mice, followed by the first of three injections (10 mg each) of the monomesyl monochloro benzoic acid mustard prodrug 4-[(2-chloroethyl)mesylamino]benzoic acid glutamide, the second given at 56 hours and the third at 72 hours. After 2 weeks the tumour was no longer detectable and the mice remain tumour free at 12 weeks. The growth of CC3 tumours in 6 untreated mice is also shown.

Attempts to introduce the prodrug into LS174T bearing mice before 120 hours after administration of A5B7 F(ab')₂-CP 50 units resulted in death of the animals and this was shown to be due to persisting enzyme activity in the blood.

Figure 2b shows accelerated clearance of 20 ug monoclonal ¹²⁵I-SB43 anticarboxypeptidase from the blood of A2G mice when 77 ug of the corresponding antigen, carboxypeptidase G2, was administered 1 hour later compared with controls which did not receive the antigen.

These data indicate that accelerated clearance of an administered antibody can be achieved by administration of a substance expressing the epitope corresponding to the binding site of the antibody.

### EXAMPLE 9

### Conjugation of TCK9 human albumin microspheres to SB43

1 mg of TCK9 human polyalbumin microspheres were derivatised with a 12.5M excess of sulpho-MBS (based on monomeric unit of 66 Kd) in a total of 1 ml phosphate buffer pH 7.8 for 2 hours at about 20°C. The mixture was centrifuged at 3000 rpm for 3 minutes and resuspended in 1 ml of buffer, and rewashed once more. 1.5 mg of ¹²⁵I labelled SB43 was thiolated by 20M excess of SPDP, according to manufacturers (Pharmacia) instructions, at about 20°C. The derivatised microspheres were centrifuged at 3000 rpm for 3 minutes, and then resuspended in the thiolated SB43 solution, the conjugation was carried out by incubating the mixture at 4°C for 72 hours.

The antibody polyalbumin conjugate was separated from the reaction mixture by centrifuging at 13000 rpm (MSE Micro centaur) for 2 minutes; the pellet was resuspended in 250 ul of sterile saline for use.

### EXAMPLE 10

Asialofetuin was given intravenously at time zero and again at 120 minutes to mice bearing LS174T xenografts. ¹²⁵¹I-A5B7-galactosylated to 10 units was administered at time +5 minutes. Mice were killed at intervals and tissues excised and radioactivity levels counted. At 24 hours the tumour to blood ratio was 27.8:1 and the tumour to liver ratio 4.2:1. All other tissues showed even more favourable ratios. It should be recognised that whereas enzyme taken up by liver is rapidly inactivated, radioactivity persists in the organ.

## Claims

1. A three component system, for use in association with one another, in the treatment of malignant diseases characterised by malignant cells, comprising: a first component which is a substance or conjugation of substances characterised by (a) one or more molecular configurations that are complementary to molecular configurations associated with malignant cells, such that the first component localises selectively at sites of malignant cells and (b) additionally by one or more catalytic sites; a second component which is able to bind to such part of the first component so as to inactivate the catalytic site of the first component and/or accelerate the clearance of the first component from the blood when the first and second components are administered clinically and a third component which is a substrate for the catalytic site on the first component, one of the end products of the catalysis being a substance which is more cytotoxic to malignant cells than the third component.

2. A system according to claim 1 wherein the first component or a part of the first component is biotinylated and in which the second component is avidin or other protein with a high affinity for binding to biotinylated proteins.

3. A system according to claim 1 wherein the first component is a conjugate of an antibody to a tumour associated antigen or a fragment thereof that includes the antigen binding site of the antibody, said antibody or fragment thereby being conjugated directly, or indirectly through a linking component to an enzyme or to an antibody or antibody fragment with catalytic functions.

4. A system according to claim 3 wherein the conjugation is effected by chemical bonding or by splicing together nucleic acid sequences that code at least for one or more antigen binding sites and one or more catalytic sites and such other sequences as are necessary to retain the vector function of the molecule and the catalytic function of the peptide when the gene product of the reconstructed nucleic acid sequence is expressed by eukaryotic or prokaryotic cells.

5. A system according to claims 3 or 4 wherein the antibody in the first component is bivalent and formed by bonding together two univalent antibody fragments, or by recombinant DNA techniques, one fragment having affinity for a tumour marker substance, the other having affinity for an enzyme.

6. A system according to claim 5 wherein the conjugate between antibody and enzyme is formed either in vitro prior to administration or in vivo by first administering the bivalent antibody, allowing time for it to localise at tumour sites and then administering the enzyme for capture by the second arm of the antibody localised at tumour sites.

7. A system according to any one of the preceding claims in which the antibody of the first component is a human immunoglobulin, or fragment thereof, having antigen binding site(s) of human origin or having antigen binding site(s) of non-human species.

8. A system according to any one of the preceding claims wherein the second component is an antibody or fragment thereof having an affinity for an antigen binding site of an antibody of the first component or the active site of an enzyme of the first component or another constituent part of the first component.

9. A system according to any one of the preceding claims wherein the second component is one causing rapid loss of enzyme activity of the first component in plasma without incurring significant loss of enzyme activity from tumour sites.

10. A system according to claim 9 wherein the second component includes covalently bound galactose residues or covalently bound residues of other sugars such as lactose or mannose, so that the second component can bind enzyme in plasma but be removed together with the enzyme or antibody-enzyme conjugate from plasma by receptors for galactose or other sugars in the liver in a period of time such that the antibody does not, enter the extravascular space of the tumour in an amount sufficient to inactivate tumour localised enzyme.

11. A system according to claim 10 wherein galactose residues in the second component are exposed by removing terminal sialic acid residues.

12. A system according to claim 1 wherein the first component is an antibody enzyme conjugate modified by addition of, or exposure of, additional galactose or other sugar residues, and is for administration in conjunction with asialofetuin, which has sufficient affinity for the corresponding sugar receptors which are involved in removing galactosylated or similar proteins from the blood, to form an asialofetuin blockade.

13. A system according to any one of claims 1 to 11 wherein the second component is conjugated to a macromolecule such as a dextran, liposome, albumin microsphere or macroglobulin with a molecular weight in excess of 500,000 Daltons or a biodegradable particle such as a blood group 0 erythrocyte so that the second component is restrained from leaving the vascular compartment by virtue of the size of the conjugate.

14. A system according to any one of the preceding claims wherein the second component is an antigen, hapten or protein construct bearing an epitope capable of binding with the first component to form complexes having accelerated clearance from plasma.

15. A system according to claim 1 or 2 wherein the first component includes a hormone or growth factor or substance other than an antibody and for which receptors capable of binding that substance exist on a tumour.

16. A system according to any one of the preceding claims wherein the first component includes an enzyme of human or non-human origin.

17. A system according to claim 16 wherein the enzyme is of non-human origin but modified by amino acid substitutions to minimise its immunogenicity in man.

18. A system according to claim 17 wherein the enzyme is conjugated to residues such as polyethylene glycol, or other polymers to reduce immunogenicity.

19. A system according to any one of the preceding claims wherein the first component is conjugated to a signal producing molecule such as a radioisotope suitable for scintigraphic imaging by gamma camera so as to confirm localisation of the first component at tumour sites.

20. A system according to any one of the preceding claims including more than one type of first component and/or more than one type of second component and/or more than one type of third component.

21. A system according to any one of the preceding claims for use in association with cyclosporin or other immunosuppresive drug or antibody to inhibit antibody response in the host to the system.

22. A system according to any one of the preceding claims for use in a method of treatment of the human or animal body by therapy.

## Patentansprüche

1. Dreikomponentensystem zur gemeinsamen Verwendung zur Behandlung von durch maligne Zellen gekennzeichneten malignen Erkrankungen, umfassend eine erste Komponente, die eine Substanz oder ein Substanzkonjugat ist, gekennzeichnet durch (a) eine oder mehrere molekulare Konfigurationen, die den mit malignen Zellen assoziierten molekularen Konfigurationen komplementär sind, so daß die erste Komponente sich selektiv an den Stellen der malignen Zellen festsetzt, und (b) zusätzlich durch eine oder mehrere katalytische Stellen; eine zweite Komponente, die an einen solchen Teil der ersten Komponente so binden kann, daß sie die katalytische Stelle der ersten Komponente inaktiviert und/oder die Ausscheidung der ersten Komponente aus dem Blut beschleunigt, wenn die erste und die zweite Komponente klinisch verabreicht werden; und eine dritte Komponente, die ein Substrat für die katalytische Stelle auf der ersten Komponente ist, wobei eines der Endprodukte der Katalyse eine Substanz ist, die für die malignen Zellen cytotoxischer ist als die dritte Komponente.

2. System nach Anspruch 1, worin die erste Komponente oder ein Teil der ersten Komponente biotinyliert ist und worin die zweite Komponente Avidin oder ein anderes Protein mit einer hohen Bindungsaffinität an biotinylierte Proteine ist.

3. System nach Anspruch 1, worin die erste Komponente ein Konjugat eines Antikörpers gegen ein tumorassoziiertes Antigen oder ein Fragment davon ist, das die Antigenbindungsstelle des Antikörpers umfaßt, wobei der Antikörper oder das Fragment dadurch direkt oder indirekt über eine Verbindungskomponente an ein Enzym oder einen Antikörper oder ein Antikörperfragment mit katalytischen Funktionen konjugiert wird.

4. System nach Anspruch 3, worin die Konjugation durch chemische Bindung oder durch Zusammenspleißen von Nucleinsäuresequenzen, die mindestens eine oder mehrere Antigenbindungsstellen und eine oder mehrere katalytische Stellen und solche anderen Sequenzen, die zur Beibehaltung der Vektorfunktion des Moleküls und der katalytischen Funktion des Peptids bei Expression des Genprodukts der rekonstruierten Nucleinsäuresequenz durch eukaryotische oder prokaryotische Zellen notwendig sind, codiert, durchgeführt wird.

5. System nach den Ansprüchen 3 oder 4, worin der Antikörper in der ersten Komponente bivalent ist und durch Verknüpfen von zwei univalenten Antikörperfragmenten oder durch rekombinante DNA-Techniken gebildet wird, wobei ein Fragment Affinität für eine Tumormarkersubstanz besitzt und das andere Affinität für ein Enzym besitzt.

6. System nach Anspruch 5, worin das Konjugat zwischen dem Antikörper und dem Enzym entweder in vitro vor der Verabreichung oder in vivo durch Verabreichen des bivalenten Antikörpers zuerst, Verstreichenlassen einer Zeit, damit er sich an den Tumorstellen festsetzt, und anschließende Verabreichung des Enzyms zum Einfang des an den Tumorstellen festgesetzten Antikörpers durch den zweiten Arm gebildet wird.

7. System nach einem der vorausgehenden Ansprüche, worin der Antikörper der ersten Komponente ein Humanimmunglobulin oder ein Fragment davon mit einer Antigenbindungsstelle/Antigenbindungsstellen menschlichen Ursprungs oder mit einer Antigenbindungsstelle/Antigenbindungsstellen einer nicht menschlichen Art ist.

8. System nach einem der vorausgehenden Ansprüche, worin die zweite Komponente ein Antikörper oder ein Fragment davon mit einer Affinität für eine Antigenbindungsstelle eines Antikörpers der ersten Komponente oder die aktive Stelle eines Enzyms der ersten Komponente oder eines anderen bildenden Teils der ersten Komponente ist.

9. System nach einem der vorausgehenden Ansprüche, worin die zweite Komponente eine ist, die einen raschen Verlust der Enzymaktivität der ersten Komponente im Plasma ohne eintretenden signifikanten Verlust der Enzymaktivität aus den Tumorstellen verursacht.

10. System nach Anspruch 9, worin die zweite Komponente covalent gebundene Galactosereste oder covalent gebundene Reste anderer Zucker, wie Lactose oder Mannose, umfaßt, so daß die zweite Komponente das Enzym im Plasma binden kann, aber zusammen mit dem Enzym oder dem Antikörperenzymkonjugat aus dem Plasma durch die Rezeptoren für Galactose oder andere Zucker in der Leber in einer Zeitspanne entfernt wird, so daß der Antikörper nicht in den extravaskulären Raum des Tumors in einer Menge, die ausreicht, das am Tumor lokalisierte Enzym zu inaktivieren, eintritt.

11. System nach Anspruch 10, worin die Galactosereste in der zweiten Komponente durch Entfernen der terminalen Sialinsäurereste freigelegt sind.

12. System nach Anspruch 1, worin die erste Komponente ein Antikörperenzymkonjugat ist, das durch Zugabe oder Freilegung von zusätzlichen Galactose- oder anderen Zuckerresten modifiziert ist und das der Verabreichung zusammen mit Asialofetuin dient, das eine ausreichende Affinität für die entsprechenden Zuckerrezeptoren besitzt, die an der Entfernung der galactosylierten oder ähnlicher Proteine aus dem Blut beteiligt sind, um eine Asialofetuinblockade zu bilden.

13. System nach einem der Ansprüche 1 bis 11, worin die zweite Komponente an ein Makromolekül, wie Dextran, Liposome, Albumin, Mikrokugeln oder Makroglobulin mit einem Molekulargewicht größer als 500.000 Dalton, oder ein biologisch abbaubares Teilchen, wie Erythrocyten der Blutgruppe 0, konjugiert ist, so daß verhindert wird, daß die zweite Komponente den Gefäßraum infolge der Größe des Konjugats verläßt.

14. System nach einem der vorausgehenden Ansprüche, worin die zweite Komponente ein Antigen, ein Hapten oder eine Proteinkonstruktion, die ein Epitop mit der Fähigkeit, die erste Komponente unter Bildung von Komplexen mit einer beschleunigten Ausscheidung aus dem Plasma zu binden, trägt, ist.

15. System nach Anspruch 1 oder 2, worin die erste Komponente ein Hormon oder einen Wachstumsfaktor oder eine Substanz, ausgenommen einen Antikörper, und für die Rezeptoren mit der Fähigkeit zur Bindung dieser Substanz auf einem Tumor vorhanden sind, umfaßt.

16. System nach einem der vorausgehenden Ansprüche, worin die erste Komponente ein Enzym menschlichen oder nicht menschlichen Ursprungs umfaßt.

17. System nach Anspruch 16, worin das Enzym nicht menschlichen Ursprungs ist, aber durch Aminosäuresubstitutionen modifiziert ist, um dessen Immunogenität beim Menschen zu minimieren.

18. System nach Anspruch 17, worin das Enzym an Reste, wie Polyethylenglycol, oder andere Polymere zur Verringerung der Immunogenität konjugiert ist.

19. System nach einem der vorausgehenden Ansprüche, worin die erste Komponente an ein signalerzeugendes Molekül, wie ein Radioisotop mit der Eignung zur szintigraphischen Abbildung durch eine Gamma-Kamera, konjugiert ist, um die Lokalisation der ersten Komponente an den Tumorstellen zu bestätigen.

20. System nach einem der vorausgehenden Ansprüche, umfassend mehr als einen Typ der ersten Komponente und/oder mehr als einen Typ der zweiten Komponente und/oder mehr als einen Typ der dritten Komponente.

21. System nach einem der vorausgehenden Ansprüche zur Verwendung zusammen mit Cyclosporin oder einem anderen immunsuppressiven Arzneimittel oder Antikörper, um die Antikörperantwort in dem Wirt gegen das System zu hemmen.

22. System nach einem der vorausgehenden Ansprüche zur Verwendung bei einem Verfahren zur therapeutischen Behandlung des menschlichen oder tierischen Körpers.

## Revendications

1. Système à trois composants, destinés à être utilisés en association mutuelle, dans le traitement d'affections malignes, caractérisées par des cellules malignes, comprenant : un premier composant qui est une substance ou une combinaison par conjuguaison de substances caractérisée par (a) une ou plusieurs configurations moléculaires qui sont complémentaires de configurations moléculaires associées à des cellules malignes, telles que le premier composant se localise sélectivement au niveau de sites de cellules malignes et (b) un ou plusieurs sites catalytiques supplémentaires ; un second composant, qui est capable de se lier à une telle partie du premier composant, de façon à inactiver le site catalytique du premier composant et/ou accélérer la clairance du premier composant à partir du sang lorsque les premier et second composants sont administrés cliniquement, et un troisième composant qui est un substrat pour le site catalytique sur le premier composant, un des produits finaux de la catalyse étant une substance qui est plus cytotoxique vis-à-vis de cellules malignes que le troisième composant.

2. Système selon la revendication 1, dans lequel le premier composant, ou une partie du premier composant, est biotinylé et dans lequel le second composant est de l'avidine ou une autre protéine ayant une grande affinité pour se lier aux protéines biotinylées.

3. Système selon la revendication 1, dans lequel le premier composant est un conjugué d'un anticorps dirigé contre un antigène associé à une tumeur ou un fragment de celui-ci, qui comprend le site de liaison à l'antigène de l'anticorps, ledit anticorps ou fragment étant ainsi conjugué directement, du indirectement par l'intermédiaire d'un composant de liaison, à une enzyme ou à un anticorps ou fragment d'anticorps ayant des fonctions catalytiques.

4. Système selon la revendication 3, dans lequel la conjugaison est réalisée par liaison chimique ou par épissage de séquences d'acides nucléiques qui codent pour au moins un ou plusieurs sites de liaison à l'antigène et un ou plusieurs sites catalytiques et d'autres séquences de ce type qui sont nécessaires pour conserver la fonction de vecteur de la molécule et la fonction catalytique du peptide lorsque le produit génique de la séquence d'acide nucléique reconstruite est exprimée dans des cellules eucaryotes ou procaryotes.

5. Système selon la revendication 3 ou 4, dans lequel l'anticorps dans le premier composant est bivalent et formé en liant ensemble deux fragments d'anticorps monovalents, ou par des techniques d'ADN recombinant, un fragment ayant une affinité pour un marqueur tumoral, l'autre ayant une affinité pour une enzyme.

6. Système selon la revendication 5, dans lequel le conjugué entre l'anticorps et l'enzyme est formé soit in vitro avant l'administration, soit in vivo en administrant en premier de l'anticorps bivalent, en lui laissant le temps de se localiser au niveau des sites tumoraux, et ensuite, en administrant l'enzyme pour la capture par le second bras de l'anticorps localisé au niveau des sites tumoraux.

7. Système selon l'une quelconque des revendications précédentes, dans lequel l'anticorps du premier composant est une immunoglobuline humaine, ou un fragment de celle-ci, ayant un ou plusieurs site(s) de liaison à l'antigène d'origine humaine ou ayant un ou plusieurs site(s) de liaison à l'antigène d'espèce non humaine.

8. Système selon l'une quelconque des revendications précédentes, dans lequel le second composant est un anticorps ou un fragment de celui-ci ayant une affinité pour un site de liaison à l'antigène d'un anticorps du premier composant ou pour le site actif d'une enzyme du premier composant ou d'une autre partie constitutive du premier composant.

9. Système selon l'une quelconque des revendications précédentes, dans lequel le second composant est un composant qui provoque une perte rapide de l'activité enzymatique du premier composant dans le plasma, sans subir de perte significative de l'activité enzymatique au niveau des sites tumoraux.

10. Système selon la revendication 9, dans lequel le second composant comprend des résidus de galactose liés par covalence ou des résidus liés par covalence d'autres sucres tels que le lactose ou le mannose, de sorte que le second composant puisse fixer l'enzyme dans le plasma mais être éliminé du plasma conjointement avec l'enzyme ou le conjugué anticorpsenzyme, par des récepteurs du galactose ou d'autres sucres dans le foie, en une période de temps telle que l'anticorps ne pénètre pas dans l'espace extravasculaire de la tumeur en une quantité suffisante pour inactiver l'enzyme localisée au niveau de la tumeur.

11. Système selon la revendication 10, dans lequel les résidus de galactose du second composant sont exposés par élimination des résidus terminaux d'acide sialique.

12. Système selon la revendication 1, dans lequel le premier composant est un conjugué anticorps-enzyme modifié par addition de, ou exposition à, des résidus supplémentaires de galactose ou d'autres sucres, et est destiné à l'administration conjointement avec de l'asialofetuine, qui présente suffisamment d'affinité pour les récepteurs de sucre correspondants qui sont impliqués dans l'élimination, à partir du sang, de protéines galactosylées ou similaires, pour former un blocage d'asialofetuine.

13. Système selon l'une quelconque des revendications 1 à 11, dans lequel le second composant est conjugué à une macromolécule telle qu'un dextrane, un liposome, une microsphère ou macroglobuline d'albumine, ayant une masse moléculaire supérieure à 500 000 daltons, ou une particule biodégradable telle qu'un érythrocyte du groupe sanguin 0, de sorte que le second composant ne peut pas quitter le compartiment vasculaire en raison de la taille du conjugué.

14. Système selon l'une quelconque des revendications précédentes, dans lequel le second composant est une structure d'antigène, d'haptène ou de protéine portant un épitope capable de se lier au premier composant pour former des complexes ayant une clairance accélérée à partir du plasma.

15. Système selon la revendication 1 ou 2, dans lequel le premier composant comprend une hormone ou un facteur de croissance ou une substance autre qu'un anticorps et pour laquelle il existe sur une tumeur des récepteurs capable de fixer cette substance.

16. Système selon l'une quelconque des revendications précédentes, dans lequel le premier composant comprend une enzyme d'origine humaine ou non humaine.

17. Système selon la revendication 16, dans lequel l'enzyme est d'origine non humaine, mais elle est modifiée par des substitutions d'acides aminés afin de minimiser son immunogénicité chez l'homme.

18. Système selon la revendication 17, dans lequel l'enzyme est conjugué à des résidus tels que du polyéthylèneglycol, ou d'autres polymères, pour réduire l'immunogénicité.

19. Système selon l'une quelconque des revendications précédentes, dans lequel le premier composant est conjugué à une molécule produisant un signal, telle qu'un isotope radioactif utilisable pour l'imagerie en scintigraphie par une caméra gamma, de façon à confirmer la localisation du premier composant au niveau des sites tumoraux.

20. Système selon l'une quelconque des revendications précédentes, comprenant plus d'un type de premier compoant et/ou plus d'un type de second composant et/ou plus d'un type de troisième composant.

21. Système selon l'une quelconque des revendications précédentes, destiné à être utilisé en association avec la cyclosporine ou d'autres médicaments ou anticorps immunosuppresseurs pour inhiber la réponse anticorps chez l'hôte vis-à-vis du système.

22. Système selon l'une quelconque des revendications précédentes, destiné à être utilisé dans un procédé de traitement thérapeutique du corps humain ou animal.
